# EUROPÄISCHE PATENTSCHRIFT

(11) **EP 3 988 049 B1**
(45) Veröffentlichungstag und Bekanntmachung des Hinweises auf die Patenterteilung: **14.06.2023**
(21) Anmeldenummer: 21196886.2
(22) Anmeldetag: 15.09.2021
(51) Int. Cl.: A61C 7/10, A61C 8/00, A61C 1/08

(54) **DISTRAKTORVORRICHTUNG**
DISTRACTOR DEVICE
DISPOSITIF DISTRACTEUR

(30) Priorität: 22.10.2020 AT 2382020
(43) Veröffentlichungstag der Anmeldung: 27.04.2022
(73) Patentinhaber: Tiger Dental GmbH, 6912 Hörbranz (AT)
(72) Erfinder: Winsauer, Julian, 6900 Bregenz (AT); Winsauer, Heinrich Friedrich, 6900 Bregenz (AT)
(74) Vertreter: Torggler & Hofmann Patentanwälte - Rankweil

(56) Entgegenhaltungen:
- DE-A1-102018 114 830
- KR-A- 20060 086 753
- KR-B1- 101 671 730
- US-B2- 9 675 796

## Beschreibung

Die vorliegende Erfindung betrifft eine Distraktorvorrichtung zum kieferorthopädischen Aufweiten eines menschlichen Kiefers, insbesondere zur Gaumennahterweiterung, wobei die Distraktorvorrichtung zumindest zwei Implantatschrauben und eine längenverstellbare Expansionseinrichtung und zumindest zwei, an der Expansionseinrichtung befestigte, Implantataufnahmehülsen für jeweils eine der Implantatschrauben aufweist, wobei der Abstand der Implantataufnahmehülsen zueinander mittels Längenverstellung der Expansionseinrichtung veränderbar ist und die Implantatschrauben jeweils einen Gewindeabschnitt zum Einschrauben der jeweiligen Implantatschraube in den Kiefer und einen Befestigungsabschnitt zur Befestigung der jeweiligen Implantatschraube in der jeweiligen Implantataufnahmehülse aufweisen.

Distraktorvorrichtungen zum kieferorthopädischen Aufweiten eines menschlichen Kiefers, insbesondere zur Gaumennahterweiterung, sind beim Stand der Technik in verschiedensten Ausgestaltungsformen bekannt. Ein Großteil der Distraktorvorrichtungen wird dabei ausschließlich an Zähnen befestigt oder kombiniert an Zähnen und Implantatschrauben. In selteneren Varianten wird die Distraktorvorrichtung ausschließlich auf Implantatschrauben befestigt. Die Implantatschrauben müssen dabei in den Kieferknochen eingeschraubt werden. Mittels der Expansionseinrichtung, welche über die Implantatschrauben an den Kieferknochen angreift, kann dann der Kiefer und insbesondere die Gaumennaht aufgeweitet werden. Hierbei wird mittels der Expansionseinrichtung der Abstand zwischen den im Kiefer verankerten Implantatschrauben während der kieferorthopädischen Behandlung nach und nach schrittweise vergrößert.

Eine gattungsgemäße Distraktorvorrichtung ist z.B. aus der BR PI 0506262-4 A bekannt. Dort sind die Befestigungsabschnitte der Implantatschrauben in den Implantataufnahmehülsen der Distraktorvorrichtung gehalten, wobei die Implantataufnahmehülsen zwischen zwei auskragenden Anschlagbereichen der Implantatschrauben eingeklemmt sind. Die Implantatschrauben müssen bei dieser Technologie in den Kiefer eingeschraubt werden, bevor die Expansionseinrichtung mit den Implantataufnahmehülsen an den Befestigungsabschnitten der Implantatschrauben befestigt werden können. In gleicher Weise muss mit der Distraktorvorrichtung gemäß der US 2009/0176190 A1 vorgegangen werden. Auch hier werden die Implantatschrauben zunächst in den Kiefer geschraubt. Die Expansionseinrichtung wird in dieser Schrift ebenfalls daran anschließend an den Befestigungsabschnitten der Implantatschrauben befestigt.

Die DE 10 2018 114 830 A1 offenbart eine gattungsgemäße Distraktorvorrichtung, bei der die Köpfe der Implantatschrauben jeweils mit einem Außengewinde in ein Innengewinde der Implantataufnahmehülsen eingeschraubt werden. Die Außengewinde an den Köpfen der Implantatschrauben haben dabei jeweils eine andere Gewindesteigung als die Gewindeabschnitte zum Einschrauben der jeweiligen Implantatschraube in den Kiefer. Weitere Distraktorvorrichtungen sind in der KR 101671730 B1, der KR 20060086753 A und der US 9,675,796 B2 gezeigt.

Ein Problem bei der oben geschilderten, beim Stand der Technik bekannten Vorgehensweise ist, dass die Implantatschrauben im Kiefer sehr exakt gesetzt werden müssen, damit hinterher eine Befestigung der Expansionseinrichtung überhaupt möglich ist.

Ausgehend vom gattungsgemäßen Stand der Technik ist es Aufgabe der Erfindung, eine Möglichkeit bereitzustellen, wie die Implantatschrauben auf einfache Art und Weise in der richtigen Positionierung in den Kiefer eingeschraubt werden können.

Ausgehend von einer Distraktorvorrichtung der oben genannten Art schlägt die Erfindung hierzu vor, dass die Implantatschrauben in zumindest einem Betriebszustand der Distraktorvorrichtung über ihre gesamte Länge vollständig durch die jeweilige Implantataufnahmehülse hindurchsteckbar sind.

Es ist somit ein Grundgedanke der Erfindung, dass der Außendurchmesser der Implantatschrauben über ihre gesamte Länge so auf den Innendurchmesser der jeweiligen Implantataufnahmehülse abgestimmt ist, dass die Implantatschraube über ihre gesamte Länge vollständig durch die jeweilige Implantataufnahmehülse hindurchgesteckt werden kann. Die Implantatschrauben und die Implantataufnahmehülsen sind also so aufeinander abgestimmt, dass die Implantatschrauben auf der einen Seite der Implantataufnahmehülse in diese eingeführt und auf der anderen Seite wieder vollständig aus ihr herausgezogen werden können. Dies ermöglicht es, dass die Implantatschrauben durch die Implantataufnahmehülsen hindurch in den Kiefer eingeschraubt werden. Die Expansionseinrichtung mit den daran befestigten Implantataufnahmehülsen kann somit als eine Schablone für das Einschrauben der Implantatschrauben im Kiefer verwendet werden. Hierdurch ist sichergestellt, dass die Implantatschrauben exakt so im Kiefer platziert und befestigt werden, wie dies für die Expansionseinrichtung benötigt wird.

Ein weiterer Vorteil der Erfindung besteht darin, dass die Expansionseinrichtung in einfacher Art und Weise von den in den Kiefer bereits eingeschraubten Implantatschrauben abgenommen werden kann, wobei die Implantatschrauben im Kiefer verbleiben. Aufgrund der erfindungsgemäßen Ausbildung ist es hierzu möglich, die Expansionseinrichtung mit den Implantataufnahmehülsen in einfacher Art und Weise auf der von den jeweiligen Gewindeabschnitten weg weisenden Seiten der Befestigungsabschnitte von den Befestigungsabschnitten und damit von den Implantatschrauben abzuziehen. Dies kann z.B. dazu genutzt werden, dass die Expansionseinrichtung in einfacher Art und Weise durch eine längere Expansionseinrichtung ausgetauscht werden kann, wenn dies im Laufe der kieferorthopädischen Behandlung aufgrund der bereits erreichten Aufweitung des Kiefers bzw. der Gaumennaht notwendig wird. Die längere Expansionseinrichtung mit ihren Implantataufnahmehülsen kann nach dem Abziehen der bisher verwendeten Expansionseinrichtung mit deren Implantataufnahmehülsen in einfacher Art und Weise wieder auf die Befestigungsabschnitte der nach wie vor im Kiefer verankerten Implantatschrauben aufgesetzt werden. Die Behandlung kann dann mit der längeren Expansionseinrichtung fortgesetzt werden.

Die erfindungsgemäße Distraktorvorrichtung könnte auch einfach als Distraktor oder als Vorrichtung bezeichnet werden. Als Distraktion bezeichnet man allgemein die kontrollierte Spreizung von Knochen in einer Spreizzone. Bei erfindungsgemäßen Distraktorvorrichtungen geht es eben um solche Distraktoren, die zum kieferorthopädischen Aufweiten des menschlichen Kiefers und insbesondere zur Gaumennahterweiterung eingesetzt werden. Die Gaumennahterweiterung ist an sich bekannt. Es handelt sich um eine kieferorthopädische Behandlung eines Schmalkiefers. Sie dient der transversalen Erweiterung des Kiefers, in der Regel des Oberkiefers. Die Gaumennahterweiterung wird bei ausgeprägten Diskrepanzen zwischen dem Ober- und Unterkieferzahnbogen angewandt und erfolgt bei ausgewachsenen Personen meist im Zusammenhang mit einer kieferchirurgischen Unterstützung.

Die Implantatschrauben sind die Schrauben der Distraktorvorrichtung, welche mit ihren Gewindeabschnitten in den Kiefer eingeschraubt werden. Sie könnten entsprechend auch als Kieferschrauben bezeichnet werden. In der Praxis werden sie häufig auch als Mini-Schrauben bezeichnet. Die Expansionseinrichtung ist der Teil der erfindungsgemäßen Distraktorvorrichtung, der für das Aufweiten des Kiefers bzw. der Gaumennaht sorgt. Die Expansionseinrichtung könnte entsprechend auch als Spreizeinrichtung bezeichnet werden. Durch ihre im Zuge der Behandlung in der Regel schrittweise erfolgende Längenverstellung wird der Abstand zwischen den Implantataufnahmehülsen und damit auch zwischen den in den Kiefer eingeschraubten Implantatschrauben vergrößert. Am Beginn der Behandlung können in der Regel nur relativ kurze Expansionseinrichtungen eingesetzt werden. Erreicht die jeweilige Expansionseinrichtung im Laufe der Behandlung ihre maximale Länge, so kann sie in der geschilderten einfachen Art und Weise durch eine längere Expansionseinrichtung ersetzt werden. Die Implantataufnahmehülsen sind die Hülsen, welche an der Expansionseinrichtung befestigt sind und der Aufnahme der Implantatschraube dienen. Sie sind also das Bindeglied zwischen der jeweiligen Implantatschraube und der Expansionseinrichtung. Der Begriff der Implantataufnahmehülsen ist allgemein aufzufassen. Man könnte auch von Implantataufnahmeausnehmungen sprechen.

Erfindungsgemäße Distraktorvorrichtungen weisen zumindest zwei Implantatschrauben und für jede Implantatschraube auch eine Implantataufnahmehülse auf. Die Implantatschrauben werden zur Behandlung in der Regel aufeinander gegenüberliegenden Seiten der Gaumennaht in den Kiefer eingeschraubt. Natürlich sind auch andere Positionierungen im Kiefer möglich, wenn nicht eine Gaumennahterweiterung sondern eine andere Behandlung im Mund durchgeführt werden soll. Wie nachfolgend geschilderte Ausführungsbeispiele zeigen, können erfindungsgemäße Distraktorvorrichtungen aber auch mehr als zwei Implantatschrauben und entsprechend auch mehr als zwei Implantataufnahmehülsen aufweisen. Dabei ist es dann in der Regel günstig, wenn jeweils die Hälfte der Implantataufnahmehülsen auf der einen Seite der Expansionseinrichtung und die andere Hälfte der Implantataufnahmehülsen auf der anderen Seite der Expansionseinrichtung angeordnet sind. Günstig ist es jedenfalls auch, wenn immer genau eine Implantatschraube in genau einer Implantataufnahmehülse gehalten bzw. befestigt wird.

In bevorzugten Varianten der erfindungsgemäßen Distraktorvorrichtung ist vorgesehen, dass die Implantatschrauben die einzige Verbindung der Distraktorvorrichtung zwischen der Expansionseinrichtung und dem Kiefer sind. In anderen Worten wird bei diesen bevorzugten Ausgestaltungsformen der Erfindung also darauf verzichtet, noch zusätzliche Befestigungen der Distraktorvorrichtung am Kiefer oder an den im Kiefer angeordneten Zähnen des Patienten vorzusehen. Man könnte in diesem Zusammenhang auch von einer ausschließlich knochengetragenen Distraktorvorrichtung sprechen.

Aufgrund der mittels der Expansionseinrichtung erzeugten Spreizung ist es denkbar, dass die Befestigungsabschnitte der Implantatschrauben ausschließlich durch Reib- bzw. Kraftschluss in den Implantataufnahmehülsen gehalten sind. Um aber auch im nicht gespannten Zustand eine sichere Befestigung der Expansionseinrichtung mit den Implantataufnahmehülsen auf den Befestigungsabschnitten der Implantatschrauben sicherzustellen, sehen bevorzugte Ausgestaltungsformen der Erfindung aber eine zusätzliche Fixierung der Implantatschrauben in den Implantataufnahmehülsen vor. Diese Fixierung weist dann einen Fixierungszustand, in dem die jeweilige Implantatschraube in der jeweiligen Implantataufnahmehülse fixiert ist, und einen Freigabezustand auf. Der Freigabezustand ist dann der Betriebszustand, in dem die jeweilige Implantatschraube über ihre gesamte Länge vollständig durch die jeweilige Implantataufnahmehülse hindurchsteckbar ist. In diesem Freigabezustand bzw. Betriebszustand ist es dann auch möglich, die Expansionseinrichtung mit den Implantataufnahmehülsen von den Befestigungsabschnitten der im Kiefer verankert bleibenden Implantatschrauben abzuziehen. Günstige Ausgestaltungsformen der Erfindung sehen somit vor, dass die Distraktorvorrichtung für zumindest eine, vorzugsweise für jede, Implantataufnahmehülse eine Fixierungseinrichtung aufweist, wobei der Befestigungsabschnitt der jeweiligen Implantatschraube in einem Fixierungszustand der Fixierungseinrichtung in der jeweiligen Implantataufnahmehülse fixierbar bzw. fixiert und in einem Freigabezustand der Fixierungseinrichtung freigegeben ist, wobei der Freigabezustand der Betriebszustand ist, in dem die jeweilige Implantatschraube über ihre gesamte Länge vollständig durch die jeweilige Implantataufnahmehülse hindurchsteckbar ist.

Wie nachfolgend noch veranschaulicht, gibt es für die Ausbildung der Fixierungseinrichtung unterschiedlichste Möglichkeiten. Eine bevorzugte Variante sieht jedoch vor, dass die Fixierungseinrichtung ein Formschlusselement und eine Formschlusselementaufnahme aufweist, wobei das Formschlusselement im Fixierungszustand in die Formschlusselementaufnahme eingreift und im Freigabezustand außerhalb der Formschlusselementaufnahme angeordnet ist. Günstig ist es dabei wiederum, wenn das Formschlusselement, vorzugsweise schwenkbar, an der jeweiligen Implantataufnahmehülse befestigt ist und die Formschlusselementaufnahme am Befestigungsabschnitt der jeweiligen Implantatschraube, vorzugsweise als umlaufende Nut, ausgebildet ist. Besonders bevorzugt ist auch vorgesehen, dass das Formschlusselement in Richtung hin zum Fixierungszustand elastisch vorgespannt ist. Durch diese elastische Vorspannung kann erreicht werden, dass der Fixierungszustand automatisch dann eintritt, wenn beim Setzen der Implantatschrauben im Kiefer, also beim Einschrauben der Gewindeabschnitte der Implantatschrauben in den Kiefer, die jeweilige Implantatschraube so weit durch die Implantataufnahmehülse hindurchgeführt bzw. hindurchgesteckt ist, dass das Formschlusselement mit der Formschlusselementaufnahme in Deckung kommt.

Für die Expansionseinrichtung sind grundsätzlich unterschiedlichste Ausgestaltungsformen denkbar, wie sie zum Teil aus dem Stand der Technik auch bekannt sind. Letztendlich muss die Expansionseinrichtung in der Lage sein, den Abstand zwischen den Implantataufnahmehülsen und damit während der kieferorthopädischen Behandlung zwischen den in den Kiefer eingeschraubten Implantatschrauben zu verändern und dann den jeweils eingestellten Abstand fest zu halten. Bevorzugte Ausgestaltungsformen der Erfindung sehen vor, dass die Expansionseinrichtung zwei Expansionsgewindestäbe und eine Expansionsmutter aufweist, wobei die Expansionsgewindestäbe jeweils an zumindest einer der Implantataufnahmehülsen fixiert und auf einander gegenüberliegenden Seiten in die Expansionsmutter eingeschraubt oder einschraubbar sind. Um die Expansionseinrichtung über einen möglichst großen Längenbereich verstellbar auszugestalten, sehen bevorzugte Varianten hierbei vor, dass einer der Expansionsgewindestäbe einen Aufnahmehohlraum aufweist, in den der andere der Expansionsgewindestäbe einführbar ist. Hierdurch kann die Expansionseinrichtung am Anfang der Behandlung sehr kurz eingestellt und im Laufe der Behandlung relativ weit ausgefahren werden. Die Gewinde der Expansionsgewindestäbe und der Expansionsmutter sind günstigerweise selbsthemmend ausgebildet. Dies bedeutet, dass eine Längenänderung der Expansionseinrichtung nur dann stattfindet, wenn diese durch Drehen der Expansionsmutter gezielt herbeigeführt wird. Ohne Betätigung der Expansionsmutter wird dann die Länge der Expansionseinrichtung gehalten und nicht verändert.

Günstigerweise ist vorgesehen, dass die oder zumindest zwei der Implantataufnahmehülsen auf einander gegenüberliegenden Seiten der Expansionseinrichtung angeordnet sind. Besonders günstig ist es, wenn Längsmittelachsen der auf einander gegenüberliegenden Seiten der Expansionseinrichtung angeordneten Implantataufnahmehülsen einen Winkel im Bereich von 100° bis 105° mit einer Längsmittelachse der Expansionseinrichtung einschließen, wobei die Expansionseinrichtung entlang ihrer Längsmittelachse längenverstellbar ist. Hierdurch kann erreicht werden, dass die durch die Implantataufnahmehülsen hindurch in den Kiefer geschraubten Implantatschrauben mit ihren Gewindeabschnitten mit einem entsprechenden Winkel auf einander gegenüberliegenden Seiten der Expansionseinrichtung voneinander weg weisen. Die Längsmittelachsen der Implantatschrauben liegen günstigerweise koaxial zu den Längsmittelachsen der jeweiligen Implantataufnahmehülsen, in denen der Befestigungsabschnitt der jeweiligen Implantatschraube befestigt ist.

Neben der Distraktorvorrichtung an sich betrifft die Erfindung auch eine Anordnung mit einer erfindungsgemäßen Distraktorvorrichtung, wobei die Anordnung zusätzlich eine Setzeinrichtung mit einer Halteplatte mit Retentionsflächen zur Befestigung der Halteplatte an im Kiefer angeordneten Zähnen und mit zumindest zwei Führungsstäben aufweist und die Distraktorvorrichtung zu den Führungsstäben korrespondierende Führungsflächen aufweist, wobei die Distraktorvorrichtung für ihre Positionierung relativ zum Kiefer entlang der Führungsstäbe verschiebbar geführt ist. Eine solche Anordnung könnte auch als ein Set bezeichnet werden. Die Setzeinrichtung ist ein Hilfsmittel für das exakte Einbauen der Distraktorvorrichtung im Kiefer. Sie sorgt dafür, dass die Distraktorvorrichtung während des Einschraubens der Implantatschrauben durch die Implantataufnahmehülsen hindurch in den Kiefer in der gewünschten Position festgehalten wird. Ist die Distraktorvorrichtung fertig eingebaut, so kann die Verriegelungseinrichtung gelöst und die Setzeinrichtung entfernt werden. Während des Einbaus der Distraktorvorrichtung wird die Setzeinrichtung mittels der Retentionsflächen und beim Stand der Technik bekannten, die Zähne abformenden Kunststoffen an den Zähnen festgehalten und damit im Kiefer exakt positioniert. Dabei kann zusätzlich ein Haftvermittler verwendet werden. Während der kieferorthopädischen Behandlung ist die Setzeinrichtung nicht mehr von Nöten und daher nicht mehr in der Mundhöhle des Patienten vorhanden.

Weitere Merkmale und Einzelheiten bevorzugter Ausgestaltungsformen der Erfindung werden nachfolgend beispielhaft anhand von erfindungsgemäßen Ausführungsvarianten geschildert. Dabei ist darauf hinzuweisen, dass die sich aus den Ausführungsvarianten ergebenden Merkmale natürlich auch in anderer Art und Weise kombiniert werden können.

Es zeigen:
Fig. 1 bis 3 verschiedene Ansichten einer ersten erfindungsgemäßen Ausführungsvariante einer Distraktorvorrichtung;
Fig. 4 eine teilweise geschnittene Darstellung dieser ersten Ausführungsvariante;
Fig. 5 und 6 Schnittdarstellungen zur Veranschaulichung der bei dieser ersten Ausführungsvariante zum Einsatz kommenden Fixierungseinrichtung;
Fig. 7 eine hier zum Einsatz kommende Implantatschraube mit einem Drehwerkzeug zum Eindrehen der Implantatschraube;
Fig. 8 eine teilweise geschnittene Darstellung zur im Kiefer eingebauten Positionierung der ersten Ausführungsvariante der erfindungsgemäßen Distraktorvorrichtung im Kiefer;
Fig. 9 die Darstellung gemäß Fig. 8 mit Setzeinrichtung;
Fig. 10 eine Draufsicht auf die auf den Zähnen angeordnete Setzeinrichtung und die daran gehaltene Distraktorvorrichtung der ersten Ausführungsvariante;
Fig. 11 die Setzeinrichtung und die Distraktorvorrichtung der ersten Ausführungsvariante in einer zu Fig. 10 gegenüberliegenden Ansicht;
Fig. 12 eine perspektivische Darstellung zu Fig. 11;
Fig. 13 und 14 Detaildarstellungen zu der in dieser Ausführungsvariante angesetzten Verriegelungseinrichtung;
Fig. 15 bis 17 eine zweite erfindungsgemäße Ausführungsvariante einer Distraktorvorrichtung in verschiedenen Ansichten;
Fig. 18 und 19 Darstellungen zu dieser zweiten erfindungsgemäßen Ausführungsvariante in einer Anordnung im Kiefer ohne und mit Setzeinrichtung;
Fig. 20 eine Draufsicht zur zweiten Ausführungsvariante der mittels einer Setzeinrichtung auf den Zähnen gehaltenen Distraktorvorrichtung;
Fig. 21 eine zu Fig. 20 gegenüberliegende Ansicht und
Fig. 22 bis 25 Beispiele für alternative Ausgestaltungsformen einer Fixierungseinrichtung.

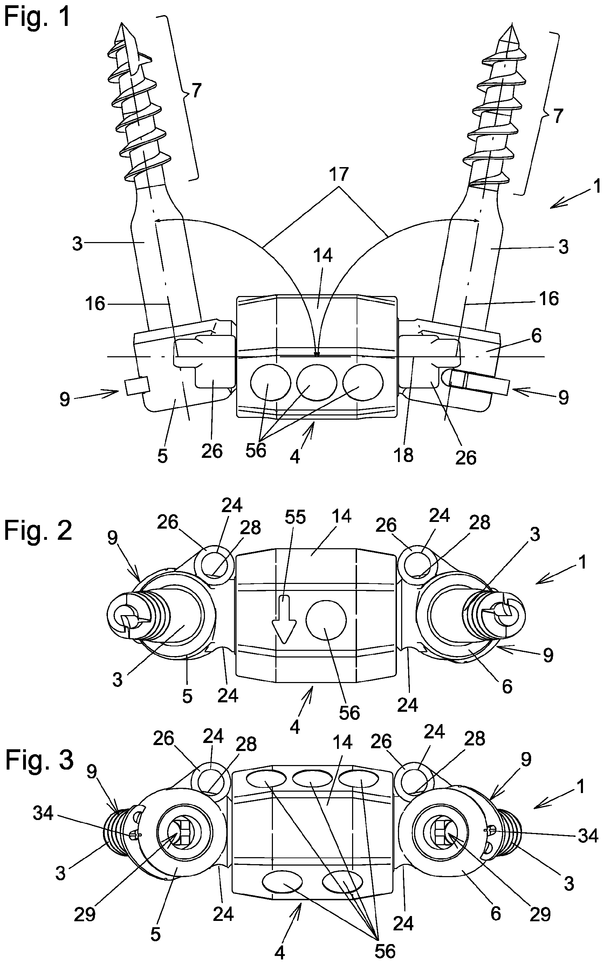

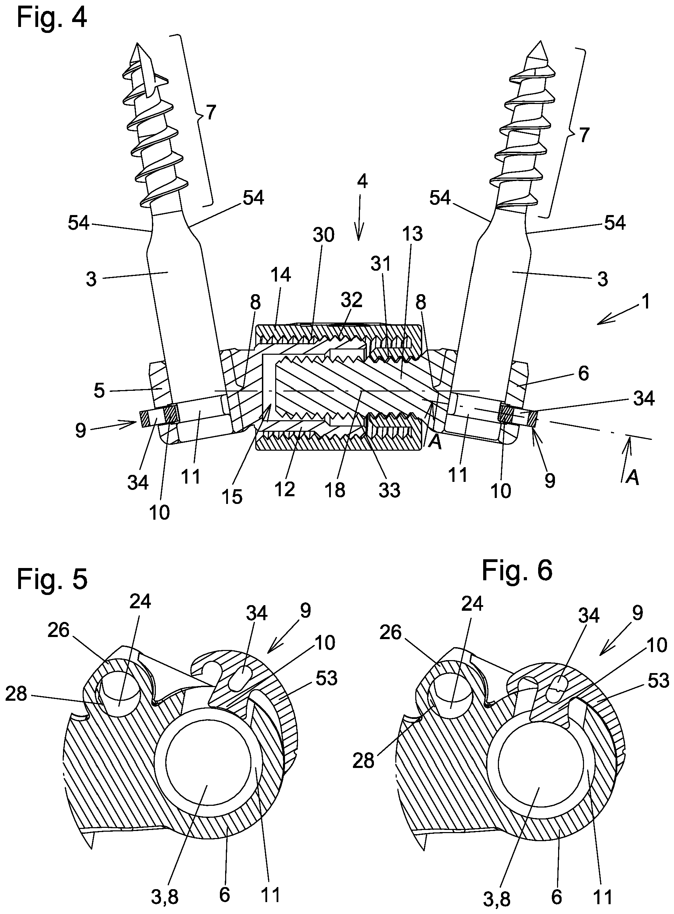

In den Fig. 1 bis 3 ist die erste erfindungsgemäße Ausführungsvariante einer Distraktorvorrichtung 1 in drei verschiedenen Ansichten gezeigt. In diesem Ausführungsbeispiel weist sie zwei Implantatschrauben 3 und eine längenverstellbare Expansionseinrichtung 4 auf. An der Expansionseinrichtung 4 sind entsprechend zwei Implantataufnahmehülsen 5 und 6 befestigt. Jede der Implantataufnahmehülsen 5 und 6 dient der Aufnahme einer der Implantatschrauben 3. Der Abstand zwischen den Implantataufnahmehülsen 5 und 6 kann mittels Längenverstellung der Expansionseinrichtung 4 verändert werden. Jede der Implantatschrauben 3 weist jeweils einen Gewindeabschnitt 7 zum Einschrauben der jeweiligen Implantatschraube 3 in den Kiefer 2 und einen Befestigungsabschnitt 8 zur Befestigung der jeweiligen Implantatsschraube 3 in der jeweiligen Implantataufnahmehülse 5 bzw. 6 auf. Erfindungsgemäß ist vorgesehen, dass die Implantatschrauben 3 in zumindest einem Betriebszustand der Distraktorvorrichtung 1 über ihre gesamte Länge vollständig durch die jeweiligen Implantataufnahmehülsen 5 bzw. 6 hindurchgesteckt werden können. Dieser Betriebszustand ist auch in diesem Beispiel der Freigabezustand der Fixierungseinrichtung 9, welcher anhand der Fig. 5 und 6 nachfolgend noch genauer erläutert wird. In der Seitenansicht gemäß Fig. 1 sieht man, dass wie hier auch realisiert, in bevorzugten Ausgestaltungsformen der Erfindung, die Längsmittelachsen 16 der aufeinander gegenüberliegenden Seiten der Expansionseinrichtung 4 angeordneten Implantataufnahmehülsen 5 und 6 jeweils einen Winkel 17 im Bereich von 100° bis 105° mit der Längsmittelachse 18 der Expansionseinrichtung 4 einschließen. Die Expansionseinrichtung 4 ist entlang ihrer Längsmittelachse 18 längenverstellbar. In den Ansichten von oben und unten gemäß der Fig. 2 und 3 ist auch gut zu sehen, dass die Distraktorvorrichtung 1 Führungsflächen 24 und Führungshülsen 26 aufweist. Auch die entsprechenden Oberflächen der Führungshülsen 26 dienen als Führungsflächen 24. In den Führungshülsen 26 sind in dieser Ausgestaltungsform aber auch schneidenartig ausgebildete Riegel 28 ausgebildet. Die Funktion der Führungsflächen 24 der Führungshülsen 26 und der Riegel 28 werden weiter hinten im Detail erläutert.

Fig. 4 zeigt eine teilweise geschnittene Darstellung, in der der innere Aufbau der hier eingesetzten Expansionseinrichtung 4 gut sichtbar dargestellt ist. Es ist hier zu sehen, dass diese bevorzugte Art der Expansionseinrichtung 4 zwei Expansionsgewindestäbe 12 und 13 und eine Expansionsmutter 14 aufweist. Die Expansionsgewindestäbe 12 und 13 sind jeweils an einer der Implantataufnahmehülsen 5 bzw. 6 fixiert und auf einander gegenüberliegenden Seiten in die Expansionsmutter 14 eingeschraubt. Im hier gezeigten Ausführungsbeispiel greift der Expansionsgewindestab 12 mit seinem Außengewinde 32 in das erste Innengewinde 30 der Expansionsmutter 14 und der Expansionsgewindestab 13 mit seinem Außengewinde 33 in das zweite Innengewinde 31 der Expansionsmutter 14 ein. In bevorzugten Ausführungsbeispielen, wie den hier gezeigten, kann dabei vorgesehen sein, dass einer der Expansionsgewindestäbe 12 einen Aufnahmehohlraum 15 aufweist, in den der andere der Expansionsgewindestäbe 13 einführbar ist. Dies erlaubt einerseits eine kurze Minimallänge der Expansionseinrichtung 4 und andererseits aber auch einen großen, maximal möglichen Verstellweg also eine große Maximallänge der Expansionseinrichtung 4. Durch Drehen der Expansionsmutter 14 um die Längsmittelachse 18 der Expansionseinrichtung 4 können die Expansionsgewindestäbe 12 und 13 in die Expansionsmutter 14 hineingeschraubt werden, wodurch der Abstand zwischen den Implantataufnahmehülsen 5 und 6 und damit auch zwischen den Implantatschrauben 3 klein eingestellt werden kann. Durch Drehen der Expansionsmutter 14 in die entgegengesetzte Richtung werden die Expansionsgewindestäbe 12 und 13 aus der Expansionsmutter 14 herausgeschraubt, wodurch sich der Abstand zwischen den auf einander gegenüberliegenden Seiten der Expansionseinrichtung 4 angeordneten Implantataufnahmehülsen 5 und 6 vergrößert. Im Zuge der kieferorthopädischen Aufweitung des Kiefers bzw. der Gaumennaht, kann so durch stückweises Vergrößern des Abstandes zwischen den Implantataufnahmehülsen 5 und 6 der Abstand zwischen den Implantatschrauben 3 vergrößert werden, um so den Kiefer bzw. die Gaumennaht nach und nach zu Weiten.

Das Drehen der Expansionsmutter erfolgt günstigerweise mittels eines hier nicht dargestellten Werkzeugs wie z.B. eines Schraubenschlüssels. Die Expansionsmutter 14 weist, wie hier dargestellt, hierzu günstigerweise eine äußere Oberfläche in Form eines Mehrkants auf, an der das entsprechende Werkzeug, insbesondere ein Schraubenschlüssel, entsprechend angreifen kann.

Bevorzugte Varianten sehen vor, dass die Expansionseinrichtung 4 eine oder mehrere Kennzeichnungen aufweist, die die momentane Stellung der Expansionsmutter 14 und/oder eine der Drehrichtungen, in denen die Expansionsmutter 14 drehbar ist, anzeigt bzw. anzeigen. In den gezeigten Ausführungsbeispielen sind hierzu auf verschiedenen Seiten der Expansionsmutter Punkte in einer unterschiedlichen Anzahl als Markierungen 56 angebracht. Diese Markierungen 56 erlauben es, die momentane Stellung der Expansionsmutter 14 zu erkennen und von anderen Stellungen zu unterscheiden. Die Pfeile 55 auf der Expansionsmutter 14 kennzeichnen eine der Drehrichtungen, in denen die Expansionsmutter 14 drehbar ist. Beides hilft, dass die Expansionsmutter 14 zum Verstellen der Länge der Expansionseinrichtung 4 in die richtige Richtung und um den richtigen Winkelbetrag gedreht wird.

In der teilweise geschnittenen Darstellung gemäß Fig. 4 sind auch weitere Details zur hier in diesem Ausführungsbeispiel realisierten Fixierungseinrichtung 9 gezeigt. Im in Fig. 4 gezeigten Fixierungszustand der Fixierungseinrichtung 9 ist der Befestigungsabschnitt 8 der jeweiligen Implantatschraube 3 in der jeweiligen Implantataufnahmehülse 5 bzw. 6 fixiert. Im in Fig. 5 dargestellten Freigabezustand der Fixierungseinrichtung 9 hingegen ist die Implantatschraube 3 bzw. ihr Befestigungsabschnitt 8 freigegeben. Der Freigabezustand ist also der Betriebszustand, in dem die jeweilige Implantatsschraube 3 erfindungsgemäß über ihre gesamte Länge vollständig durch die jeweilige Implantataufnahmehülse 5 bzw. 6 hindurchsteckbar ist. Der Fixierungszustand und der Freigabezustand dieses ersten Ausführungsbeispiels einer Fixierungseinrichtung 9 ist in den Schnittdarstellungen gemäß Fig. 5 und 6 gezeigt. Die Fig. 5 und 6 zeigen jeweils Schnitte in der in Fig. 4 eingezeichneten Schnittebene A-A. Fig. 5 zeigt den Freigabezustand, Fig. 6 den Fixierungszustand. Die Fixierungseinrichtung 9 dieses Ausführungsbeispiels weist ein Formschlusselement 10 und eine Formschlusselementaufnahme 11 auf, wobei das Formschlusselement 10 im Fixierungszustand gemäß Fig. 6 in die Formschlusselementaufnahme 11 eingreift und im Freigabezustand gemäß Fig. 5 außerhalb der Formschlusselementaufnahme 11 angeordnet ist. Dies kann grundsätzlich in verschiedensten Ausgestaltungsformen realisiert werden. In der hier gezeigten Variante ist das Formschlusselement 10 Teil eines schwenkbaren Armes 53, welcher schwenkbar an der jeweiligen Implantataufnahmehülse 5 bzw. 6 befestigt ist. Die Formschlusselementaufnahme 11 ist in diesem Ausführungsbeispiel am Befestigungsabschnitt 8 der jeweiligen Implantatschraube 3 ausgebildet. In der gezeigten Variante handelt es sich bei der Formschlusselementaufnahme 11 um eine ringförmig umlaufende Nut im Befestigungsabschnitt 8 der jeweiligen Implantatschraube 3. In bevorzugten Ausgestaltungsformen wie den hier gezeigten, ist das Formschlusselement 10 in Richtung hin zum Fixierungszustand gemäß Fig. 6 elastisch vorgespannt. Hierdurch wird erreicht, dass das Formschlusselement 10 immer dann automatisch in die Formschlusselementaufnahme 11 einrastet, wenn die Formschlusselementaufnahme 11 durch entsprechend weites Einführen des Befestigungsabschnitts 8 der Implantatschraube 3 in die jeweilige Implantataufnahmehülse 5 bzw. 6 in Deckung gebracht ist. Um die Fixierungseinrichtung 9 ausgehend von dem Fixierungszustand gemäß Fig. 6 in den Freigabezustand gemäß Fig. 5 zu bringen, wird der Schwenkarm 53 entgegen seiner elastischen Vorspannung so weit ausgelenkt, dass das Formschlusselement 10 nicht mehr in die Formschlusselementaufnahme 11 eingreift. Das Auslenken des schwenkbaren Arms 53 kann mittels eines Werkzeuges ausgeführt werden. Im hier gezeigten Ausführungsbeispiel ist hierzu ein Werkzeugeingriff 34 vorgesehen, in das ein Werkzeug wie z.B. ein entsprechend dünner Stab eingeführt werden kann, um den schwenkbaren Arm 53 entsprechend auszulenken.

In den Fig. 5 und 6 ist ein Schnitt durch die Implantataufnahmehülse 6 gezeigt. Im hier realisierten ersten Ausführungsbeispiel ist an der anderen Implantataufnahmehülse 5 genau derselbe Mechanismus mit einer entsprechenden Fixiereinrichtung 9 ausgebildet. Auf eine separate Darstellung der Fixiereinrichtung 9 im Bereich der Implantataufnahmehülse 5 kann entsprechend verzichtet werden.

Auch wenn dies, wie eingangs bereits erläutert, nicht zwingend vorgesehen sein muss, so ist es doch günstig, wenn, wie hier gezeigt, an jeder Implantataufnahmehülse 5 bzw. 6 eine entsprechende Fixierungseinrichtung 9 vorgesehen ist. Befinden sich alle Fixierungseinrichtungen 9 in ihrem Fixierungszustand, so ist die Expansionseinrichtung 4 sicher an den Implantataufnahmeschrauben 3 fixiert. Befinden sich hingegen alle Fixierungseinrichtungen 9 in ihrem Freigabezustand, so kann die Expansionseinrichtung samt Implantataufnahmehülsen 5 und 6 von den im Kiefer 2 eingeschraubten Implantatschrauben 3 abgezogen werden, wie dies weiter hinten noch im Detail erläutert wird.

Beim Einschrauben der Implantatschrauben 3 durch die jeweilige Implantataufnahmehülse 5 bzw. 6 hindurch in den Kiefer 2 hinein erfolgt günstigerweise ein automatisches Auslenken des Formschlusselements 10. Im hier gezeigten Ausführungsbeispiel ist dies dann der Fall, wenn die zwischen dem Gewindeabschnitt 7 und dem Befestigungsabschnitt 8 an der jeweiligen Implantatschraube 3 ausgebildeten Schrägflächen 54 auf das Formschlusselement 10 treffen und die Schraube dann weiter in den Kiefer eingeschraubt wird. Ist dann die in Fig. 4 gezeigte Endstellung gezeigt, so rastet das Formschlusselement 10 dieser Art der Fixierungseinrichtung 9 aufgrund seiner elastischen Vorspannung automatisch in die jeweilige Formschlusselementaufnahme 11 ein, womit dann der Fixierungszustand automatisch erreicht wird.

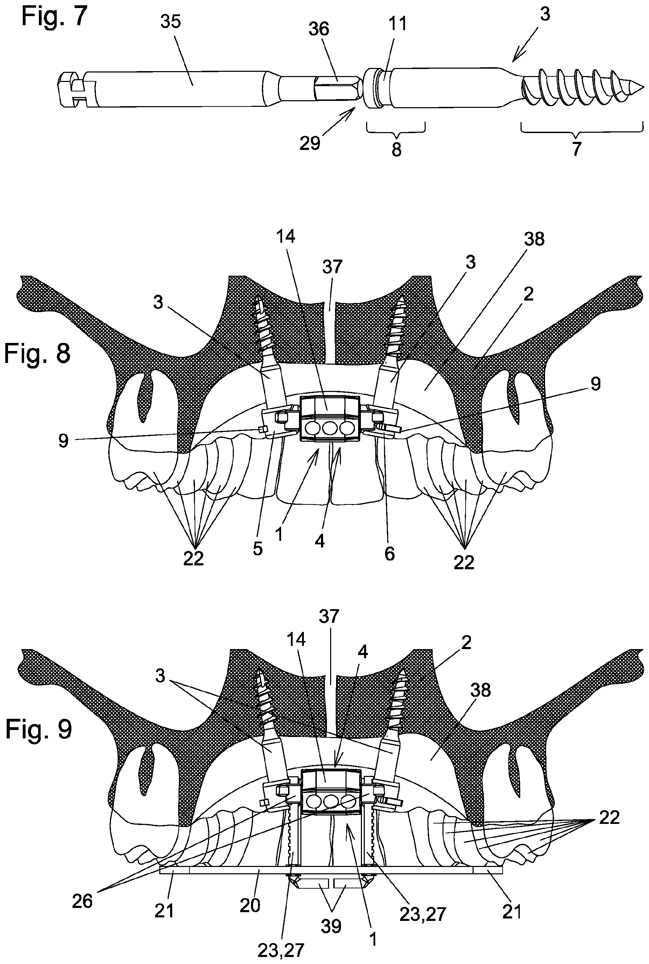

Fig. 7 zeigt eine der hier eingesetzten Implantatschrauben 3. Diese weist an ihrem, dem Gewindeabschnitt 7 gegenüberliegenden Ende des Befestigungsabschnitts 8 eine Werkzeugaufnahme 29 auf, in die das in Fig. 7 beispielhaft gezeigte Drehwerkzeug 35 mit seinem hier beispielhaft dargestellten Mehrkant 36 eingreifen kann, um so mittels des Drehwerkzeugs 35 die Implantatschraube 3 in den Kiefer 2 hineinzuschrauben oder aus diesem herauszuschrauben. In bevorzugten Ausgestaltungsformen, wie der hier gezeigten, ist die Werkzeugaufnahme 29 bevorzugt innerhalb des Befestigungsabschnitts 8 bzw. Implantatschraube 3 angeordnet. Es kann sich bei der Werkzeugsaufnahme 29 wie in diesem Ausführungsbeispiel um einen entsprechend ausgeformten Mehrkant handeln.

In dem hier gezeigten Ausführungsbeispiel sind die Aufnahmehohlräume der Implantaufnahmehülsen 5 und 6, durch die die Implantatschrauben 3 hindurchsteckbar sind, abgesehen von dem Bereich, in dem die Fixierungseinrichtungen 9 angeordnet sind, kreiszylinderförmig ausgebildet. Entsprechend ist in den hier gezeigten Ausführungsbeispielen der Befestigungsabschnitt 8, abgesehen von der Formschlusselementaufnahme 11, an seiner äußeren Oberfläche ebenfalls kreiszylinderförmig ausgebildet. Dies muss aber nicht zwingend so sein. Es reicht aus, wenn die Form des Aufnahmehohlraums in den Implantataufnahmehülsen 5 und 6 so an die Außenkontur der Implantatschraube 3 angepasst ist, dass die Implantatschraube 3 eben erfindungsgemäß über ihre ganze Länge durch die jeweilige Implantataufnahmehülse 5 bzw. 6 hindurchgesteckt werden kann.

Fig. 8 zeigt in einem Schnitt durch einen mit Zähnen 22 besetzten menschlichen Kiefer 2 den fertig eingebauten Zustand der erfindungsgemäßen Distraktorvorrichtung 1 der ersten Ausführungsvariante, in dem die kieferorthopädische Behandlung zur Weitung des Kiefers 2 und damit auch der Gaumennaht 37 durch schrittweises Vergrößern der Länge der Expansionseinrichtung 4 erfolgen kann. Die Fixierungseinrichtungen 9 befinden sich dabei in ihrem Fixierungszustand. Die Implantatschrauben 3 sind durch die Implantataufnahmehülsen 5 und 6 und die Gingiva 38 hindurch entsprechend weit in den Kiefer 2 eingeschraubt, sodass die Distraktorvorrichtung 1 ausreichend fest mit dem Kiefer 2 verbunden ist. Die Gaumennaht 37 befindet sich zwischen den beiden Implantatschrauben 3, sodass ein entsprechendes Vergrößern der Länge der Expansionseinrichtung 4 zu einer entsprechenden Aufweitung der Gaumennaht 37 und damit des Kiefers 2 in transversaler Richtung führt. Hat die bislang verwendete Expansionseinrichtung 4 ihre maximal mögliche Länge erreicht und soll sie zum weiteren Aufweiten der Gaumennaht 37 bzw. des Kiefers 2 durch eine längere Expansionseinrichtung 4 ersetzt werden, so können bei der gezeigten Distraktorvorrichtung 1 gemäß der Erfindung die Implantatschrauben 3 im Kiefer 2 verbleiben. Es werden lediglich die Fixierungseinrichtungen 9 geöffnet, sodass die Expansionseinrichtung 4 mitsamt den Implantataufnahmehülsen 5 und 6 von den Befestigungsabschnitten 8 der jeweiligen Implantatschrauben 3 in Richtung vom Kiefer 2 bzw. den Gewindeabschnitten 7 der Implantatschrauben 3 weg abgezogen werden kann. In Ausführungsbeispielen, wie den hier gezeigten, in denen die Längsmittelachsen 16 der Implantataufnahmehülsen 5 und 6 einen entsprechend großen Winkel 17 zu der Längsmittelachse 18 der Expansionseinrichtung 4 aufweisen, kann dabei das Abziehen der Expansionseinrichtung von den Befestigungsabschnitten 8 der Implantatschrauben 3 durch ein entsprechendes Drehen der Expansionsmutter 14 und damit ein Verkürzen der Expansionseinrichtung 4 vereinfacht werden. Ob diese unterstützende Maßnahme zum Abziehen der Expansionseinrichtung 4 samt Implantataufnahmehülsen 5 und 6 von den Implantatschrauben 3 notwendig ist oder nicht, hängt davon ab, in welchem Winkel 17 die Implantataufnahmehülsen 5 und 6 relativ zur Expansionseinrichtung 4 angeordnet sind.

Wie eingangs bereits erläutert, betrifft die Erfindung nicht nur eine Distraktorvorrichtung 1 an sich, sondern auch eine Anordnung bzw. ein Set mit einer solchen Distraktorvorrichtung 1 und einer Setzeinrichtung 19, mit der das lagerichtige Setzen bzw. der lagerichtige Einbau der Distraktorvorrichtung 1 am Kiefer 2 erleichert wird. In den Fig. 9 bis 12 ist für das erste Ausführungsbeispiel die Distraktorvorrichtung 1 zusammen mit der Setzeinrichtung 19 gezeigt. Fig. 9 zeigt dabei den Zustand am Ende des Setz- bzw. Einbauvorgangs, bei dem die Distraktorvorrichtung 1 bereits mittels der Implantatschrauben 3 fertig im Kiefer 2 gesetzt bzw. eingebaut, die Setzeinrichtung 19 aber noch nicht entfernt ist. Ausgehend von diesem Zustand gemäß Fig. 9 kann die Setzeinrichtung 19 dann abgenommen werden, sodass sich das Bild gemäß Fig. 8, also der Zustand, in dem die kieferorthopädische Behandlung durchgeführt wird, ergibt.

Die Setzeinrichtung 19 weist eine Halteplatte 20 mit Retentionsflächen 21 auf. Mittels der Retentionsflächen 21 kann die Halteplatte 20 an den im Kiefer 2 angeordneten Zähnen 22 temporär für den Setzvorgang befestigt werden. Die Retentionsflächen 21 werden dabei mittels eines entsprechenden, an sich beim Stand der Technik bekannten, die Zähne abformenden Kunststoffes an den Zähnen festgehalten und damit im Kiefer exakt positioniert. Dabei kann zusätzlich ein Haftvermittler verwendet werden. Diese Kunststoffe ermöglichen einerseits eine ausreichende Befestigung der Setzeinrichtung 19 während es Setzvorgangs und andererseits aber ein anschließendes Ablösen der Setzeinrichtung 19 von den Zähnen 22. An der Halteplatte 20 weist die Setzeinrichtung 19 Führungsstäbe 23 auf. Die Distraktorvorrichtung 1 kann mit den zu den Führungsstäben 23 korrespondierenden Führungsflächen 24 zum Positionieren der Distraktorvorrichtung 1 relativ zum Kiefer 2 entlang der Führungsstäbe 23 verschoben werden. Zusätzlich weist die Setzeinrichtung 19 auch eine Verriegelungseinrichtung 25 auf, welche dem lösbaren Verriegeln der Distraktorvorrichtung 1 in der relativ zum Kiefer 2 eingestellten Position an der Setzeinrichtung 19 dient. An der Distraktorvorrichtung 1 sind Führungsflächen 24 ausgebildet, mit denen die Distraktorvorrichtung 1 an den Führungsstäben 23 geführt, in Richtung von der Halteplatte 20 weg, also in Richtung zur Gingiva 38 hin und in die entgegengesetzte Richtung geführt verschoben werden kann. So kann letztendlich der gewünschte Abstand zwischen der Expansionseinrichtung 4 und der Gingiva 38 eingestellt werden. Die optimale Positionierung der Distraktorvorrichtung 1 in den dazu orthogonalen Richtungen parallel zur Okklusionsebene der Zähne 22 erfolgt durch das entsprechende Positionieren der Halteplatte 20 mit ihren Retentionsflächen 21 auf den Zähnen 22. Natürlich können dabei für verschieden große Gebisse verschieden große bzw. verschieden geformte Halteplatten 20 zur Verfügung gestellt werden.

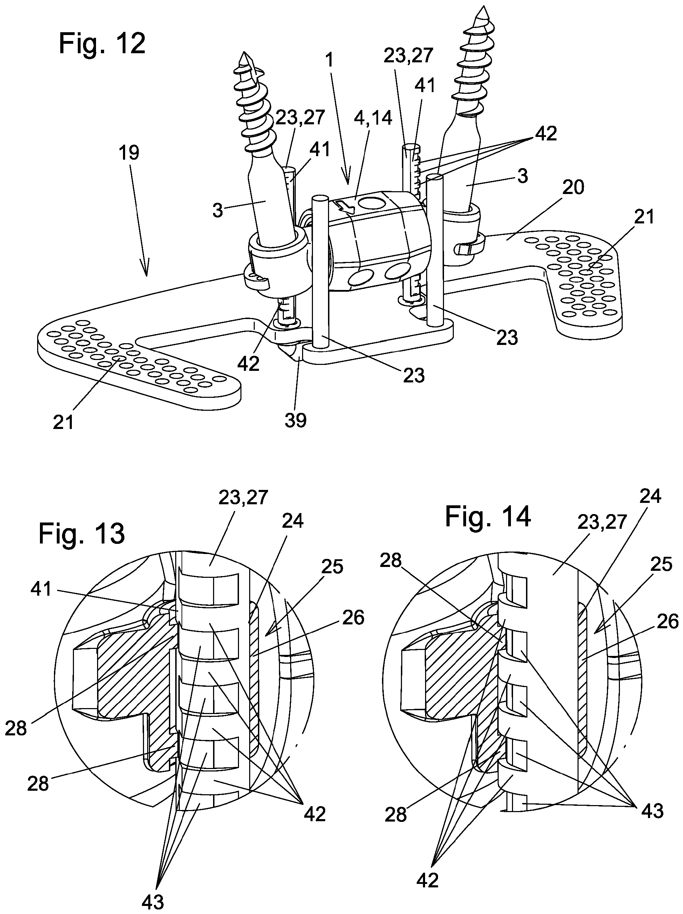

Die in diesem Ausführungsbeispiel realisierte Art der Verriegelungseinrichtung 25, mit der die Distraktorvorrichtung 1 in der entsprechenden Entfernung von der Halteplatte 20 an der Setzeinrichtung 19 lösbar verriegelt werden kann, wird nun anhand der Fig. 12 bis 14 erläutert. Die Fig. 13 und 14 zeigen teilweise geschnittene Darstellungen im Bereich der Führungshülse 26. Im hier gezeigten Ausführungsbeispiel sind in jeder Führungshülse 26 zwei Riegel 28 vorgesehen. Dies muss aber nicht so sein. Grundsätzlich reicht auch ein Riegel 28 aus. Natürlich können auch mehr als zwei Riegel 28 vorgesehen sein.

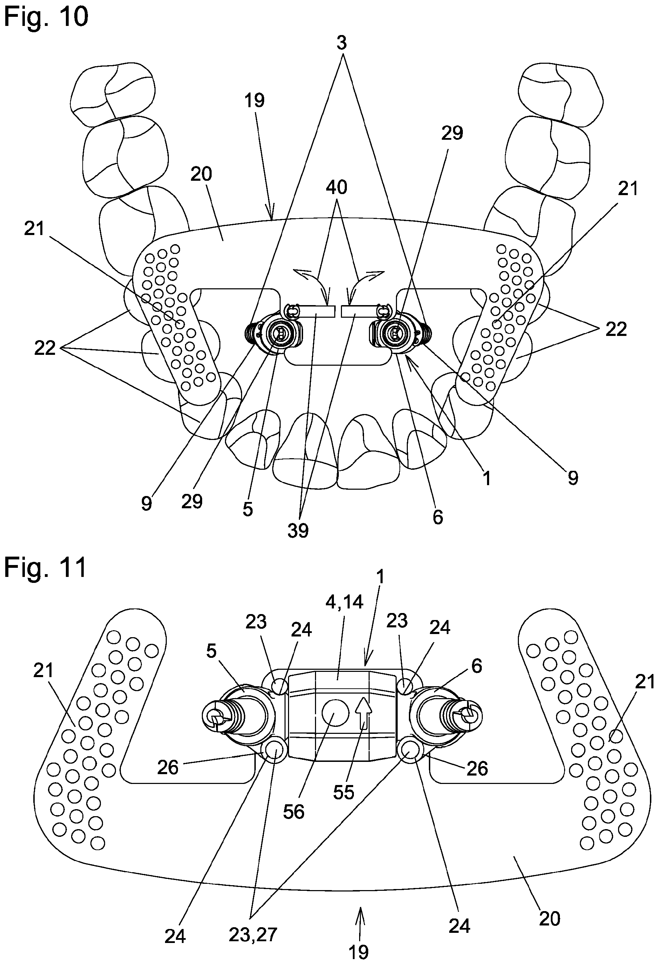

In der hier gezeigten Ausführungsvariante sind zwei der vier insgesamt vorhandenen Führungsstäbe 23 gleichzeitig als Verriegelungsstäbe 27 ausgeführt. Sie weisen jeweils über einen gewissen Längenabschnitt eine Verzahnung 42 und benachbart dazu eine Abflachung 41 auf. Die Verzahnung 42 wird durch eine Abfolge von Zähnen und dazwischen ausgebildeten Zwischenräumen 43 gebildet. Die als Verriegelungsstäbe 27 ausgebildeten Führungsstäbe 23 sind jeweils um ihre Längsachse mittels der Griffe 39 in den in Fig. 10 dargestellten Schwenkrichtungen 40 schwenkbar an der Halteplatte 20 befestigt. Die Führungshülsen 26, in die diese Verriegelungsstäbe 27 eingeführt werden, weisen einerseits die Führungsflächen 24 zur Führung des jeweiligen, als Verriegelungsstab 27 ausgebildeten Führungsstabes 23 und andererseits aber die an der Führungshülse 26 fix ausgebildeten Riegel 28 auf. Soll die Distraktorvorrichtung 1 entlang der Führungsstäbe 23 und der Verriegelungsstäbe 27 verschoben werden können, so werden die Verriegelungsstäbe 27 mittels der Griffe 39 in die Stellung gemäß Fig. 13 gebracht, in der die Riegel 28 im Bereich der Abflachung 41 angeordnet sind. In dieser Stellung kann die Distraktorvorrichtung 1 entlang der Führungsstäbe 23 und der Verriegelungsstäbe 27 verschoben bzw. die Setzeinrichtung 19 ganz von der Distraktorvorrichtung 1 abgezogen werden. Zum Verriegeln der hier beispielhaft gezeigten Verriegelungseinrichtung 25 werden die Griffe 39 dann in die entsprechende Schwenkrichtung 40 geschwenkt, sodass die Verzahnung 42 am Verriegelungsstab 27 in Eingriff mit dem zumindest einen Riegel 28 in der Führungshülse 26 kommt. Dies ist dann der Fall, wenn der Riegel 28 durch entsprechendes Drehen des Verriegelungsstabes 27 in einen Zwischenraum 43 der Verzahnung 42 gelangt. Ist diese, in Fig. 14 gezeigte Stellung erreicht, so ist die Distraktorvorrichtung 1 an der Setzeinrichtung 19 verriegelt und kann nicht mehr entlang der Führungsstäbe 23 und der Verriegelungsstäbe 27 verschoben werden.

Für die Ausbildung der Verriegelungseinrichtung 25 gibt es natürlich auch alternative Möglichkeiten. So kann bei entsprechender Ausbildung der Führungshülsen 26 und der Verriegelungsstäbe 27 auch eine rein klemmende Befestigung der Distraktorvorrichtung 1 an den Verriegelungsstäben 27 realisiert werden. Natürlich gibt es darüber hinaus aber auch noch andere Möglichkeiten. Zum Beispiel könnte die Reibung zwischen den Führungsflächen 24 und den Führungsstäben 23 so groß sein, dass sie zum Festhalten der Distraktorvorrichtung 1 in einer Relativposition zur Setzeinrichtung 19 ausreicht.

Zum Setzen bzw. zum Einbau der erfindungsgemäßen Distraktorvorrichtung 1 in den Kiefer 2 wird zunächst die Expansionseinrichtung 4 der Distraktorvorrichtung 1 mittels seiner Führungsflächen 24 auf die Führungsstäbe 23 und die Verriegelungsstäbe 27 geschoben, wobei sich die Verriegelungseinrichtung 25 in der geöffneten Stellung befindet. Befindet sich die Expansionseinrichtung 4 in der gewünschten Entfernung von der Halteplatte 20, so wird die Verriegelungseinrichtung 25 durch entsprechendes Schwenken der Griffe 39 in die verriegelte Stellung gebracht, sodass der Abstand zwischen der Expansionseinrichtung 4 und der Halteplatte 20 fixiert ist. Anschließend wird die Setzeinrichtung 19 mit ihren Retentionsflächen 21 im Kiefer 2 an den Zähnen 22 ausgerichtet und mit dem genannten, die Zähne abformenden Kunststoff befestigt. Dann kann geprüft werden, ob sich die Expansionseinrichtung 4 im optimalen Abstand von der Gingiva 38 bzw. dem Kiefer 2 befindet. Wenn nicht, kann durch entsprechendes Öffnen der Verriegelungseinrichtung 25 und Verschieben der Expansionseinrichtung 4 entlang der Führungsstäbe 23 und Verriegelungsstäbe 27 nachjustiert werden. Ist dann die optimale Position erreicht, so werden die Verriegelungseinrichtungen 25 wieder in ihre verriegelte Stellung gebracht. Ist dies erreicht, so befindet sich die Expansionseinrichtung 4 mit ihren Implantataufnahmehülsen 5 und 6 in der optimalen Position relativ zum Kiefer 2. Nun können die Implantatschrauben 3 durch die jeweilige Implantataufnahmehülse 5 und 6 hindurchgeführt und mit ihrem Gewindeabschnitt 7 in den Kiefer 2 eingeschraubt werden. Die Expansionseinrichtung 4 mit ihren Implantataufnahmehülsen 5 und 6 dient dabei als eine Art Schablone bzw. Führung, die dafür sorgt, dass die Implantatschrauben 3 genau in der Position und in dem Winkel in den Kiefer 2 eingeschraubt werden, wie dies für die nachfolgende Behandlung gewünscht ist. Die Implantatschrauben 3 werden so weit in den Kiefer 2 eingeschraubt, bis die Fixierungseinrichtung 9 die sich dann in den Implantataufnahmehülsen 5 und 6 befindenden Befestigungsabschnitte 8 fixiert. Im gezeigten Ausführungsbeispiel ist dies die Stellung, in der das Formschlusselement 10 der jeweiligen Fixierungseinrichtung 9 in die Formschlusselementaufnahme 11 der jeweiligen Implantatschraube 3 einrastet. Ist dies der Fall, so ist die Stellung gemäß Fig. 9 erreicht. Nun kann die Verriegelungseinrichtung 25 durch entsprechendes Schwenken der Griffe 39 und damit der Verriegelungsstäbe 27 in ihre entriegelte Stellung gebracht werden, sodass dann die Retentionsflächen 21 der Halteplatte 20 von den Zähnen 22 gelöst und die gesamte Setzeinrichtung 19 von der Distraktorvorrichtung 1 abgezogen werden kann. Ist dies vollbracht, so ist die Situation gemäß Fig. 8 erreicht, in der dann die kieferorthopädische Behandlung durch stückweises Vergrößern der Länge der Expansionseinrichtung 4 und damit des Abstandes zwischen den Implantatschrauben 3 durchgeführt wird. Ist dann im Laufe der Behandlung die maximale Länge der bis dann verwendeten Expansionseinrichtung 4 erreicht, so kann diese nach Lösen der Fixierungseinrichtung 9 in Richtung weg vom Kiefer 2 von den Befestigungsabschnitten 8 der Implantatschrauben 3 abgezogen und durch eine entsprechend längere Expansionseinrichtung 4 ersetzt werden. Zur Bereitstellung einer längeren Expansionseinrichtung 4 kann es z.B. auch ausreichen, wenn man die bislang verwendete Expansionsmutter 14 durch eine längere Expansionsmutter 14 ersetzt und die bereits vorher verwendeten Implantataufnahmehülsen 5 und 6 zusammen mit der längeren Expansionsmutter 14 als längere Expansionseinrichtung 4 weiter verwendet.

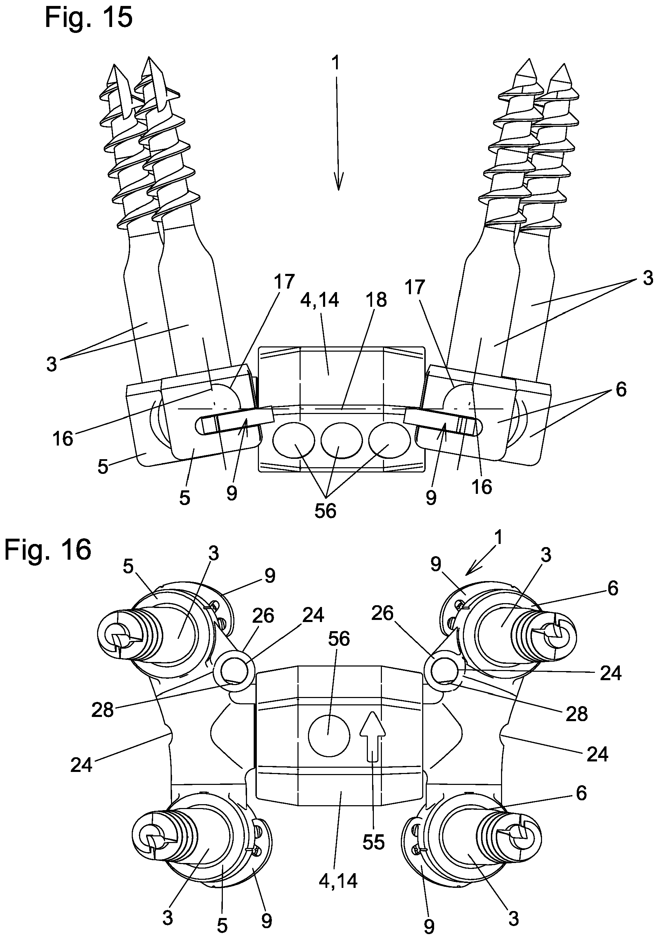

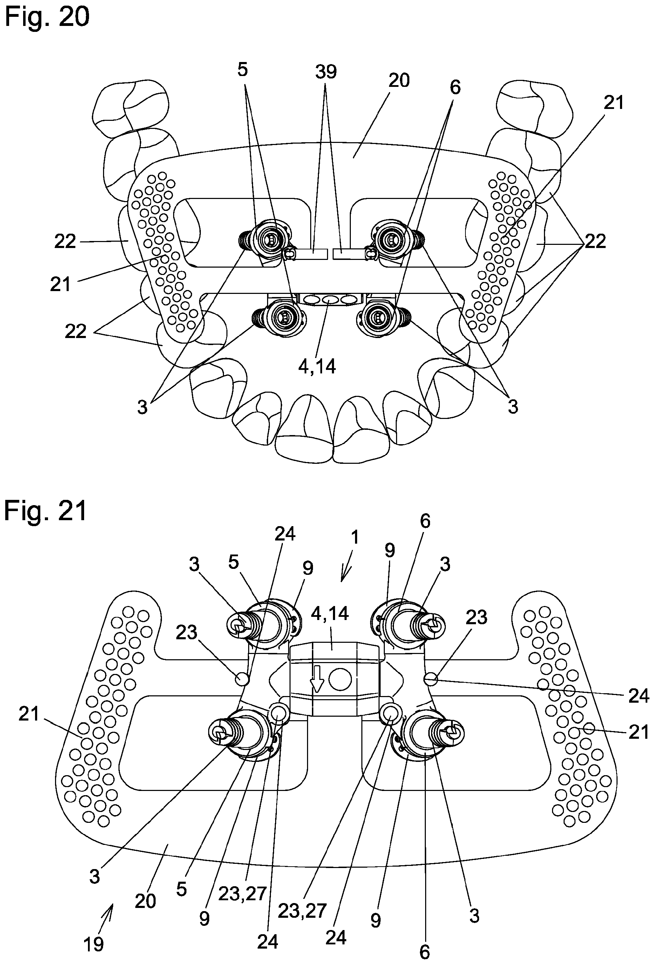

Die Fig. 15 bis 21 zeigen ein zweites erfindungsgemäßes Ausführungsbeispiel sowohl einer Distraktorvorrichtung 1 als auch einer Anordnung aus Distraktorvorrichtung 1 und Setzeinrichtung 19. Dieses zweite Ausführungsbeispiel dient der Veranschaulichung, dass die Distraktorvorrichtung 1 auch mehr als zwei Implantatschrauben 3 und Implantataufnahmehülsen 5 und 6 aufweisen kann. Im zweiten Ausführungsbeispiel sind dies vier Implantatschrauben 3 und entsprechend auch vier Implantataufnahmehülsen 5 und 6. Wie eingangs bereits erläutert, ist es dabei günstig, wenn wie hier auch realisiert, die eine Hälfte der Implantatschrauben 3 und der Implantataufnahmehülsen 5 auf der einen Seite und die andere Hälfte der Implantatschrauben 3 und der Implantataufnahmehülsen 6 auf der anderen Seite der Expansionseinrichtung 4 ausgebildet bzw. angeordnet sind. Abgesehen von diesem Unterschied zur ersten Ausführungsvariante gemäß der Fig. 1 bis 14 ist diese zweite erfindungsgemäße Ausführungsvariante im Wesentlichen so wie die erste Ausführungsvariante ausgebildet, sodass nachfolgend nur noch auf die Unterschiede eingegangen wird. Ansonsten gelten die obigen Ausführungen zur ersten Ausführungsvariante. Insbesondere die Implantatschrauben 3, die Expansionseinrichtung 4, die Implantataufnahmehülsen 5 und 6, deren Fixierungseinrichtung 9 sowie die Verriegelungseinrichtung 25 der Setzeinrichtung 19 sind identisch zum ersten Ausführungsbeispiel ausgeführt, sodass auf die dortigen Erläuterungen verwiesen werden kann. Auch der in Fig. 15 eingezeichnete Winkel 17 zwischen den Längsmittelachsen 16 der Implantataufnahmehülsen 5 und 6 der Längsmittelachse 18 der Expansionseinrichtung 4, ist wie im ersten Ausführungsbeispiel ausgebildet. Die Längsmittelachsen 18 der jeweils auf einer Seite der Expansionseinrichtung 4 angeordneten Implantataufnahmehülsen 5 hingegen sind parallel zueinander ausgerichtet. Das Gleiche gilt für die auf der anderen Seite angeordneten Implantataufnahmehülsen 6.

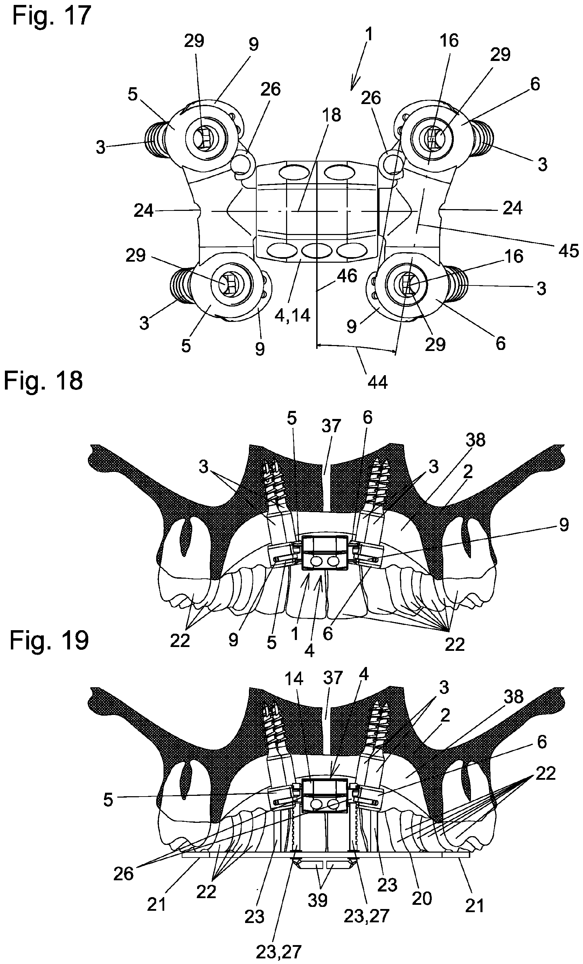

Hierdurch verlaufen die beiden durch die Implantataufnahmehülsen 5 hindurchgeführten Implantatschrauben 3 parallel zueinander und die beiden durch die Implantataufnahmehülsen 6 hindurchgeführten Implantatschrauben 3 ebenfalls parallel zueinander. In Fig. 17 ist noch der Winkel 44 eingezeichnet. Der Winkel 44 liegt günstigerweise in einem Bereich zwischen 10° und 15°. Es handelt sich dabei um den Winkel 44 zwischen einer Gerade 45, welche durch die Längsmittelachsen 16 der auf einer Seite der Expansionseinrichtung 4 angeordneten Implantataufnahmehülsen 5 oder 6 verlaufen, und einer Orthogonalen 46 auf die Längsmittelachse 18 der Expansionseinrichtung 4.

Die Fig. 18 bis 21 zeigen dieses zweite erfindungsgemäße Ausführungsbeispiel in zu den Fig. 8 bis 11 analogen Darstellungen. Um Wiederholungen zu vermeiden, wird lediglich darauf hingewiesen, dass der Setzvorgang bei diesem zweiten erfindungsgemäßen Ausführungsbeispiel und damit der Einbau der Distraktorvorrichtung 1 mittels der Setzeinrichtung 19 in analoger Art und Weise wie beim ersten Ausführungsbeispiel durchgeführt wird. Die eigentliche kieferorthopädische Behandlung zur Aufweitung des Kiefers 2 bzw. der Gaumennaht 37 erfolgt ebenfalls wie im ersten Ausführungsbeispiel. Das Gleiche gilt für den Fall, dass im Laufe der Behandlung eine Expansionseinrichtung 4 durch eine längere Expansionseinrichtung 4 ersetzt werden muss.

Die Verwendung von erfindungsgemäßen Distraktorvorrichtungen 1 mit mehr als zwei Implantatschrauben 3 ist vor allem dann angezeigt, wenn ausgewachsene Personen behandelt werden sollen.

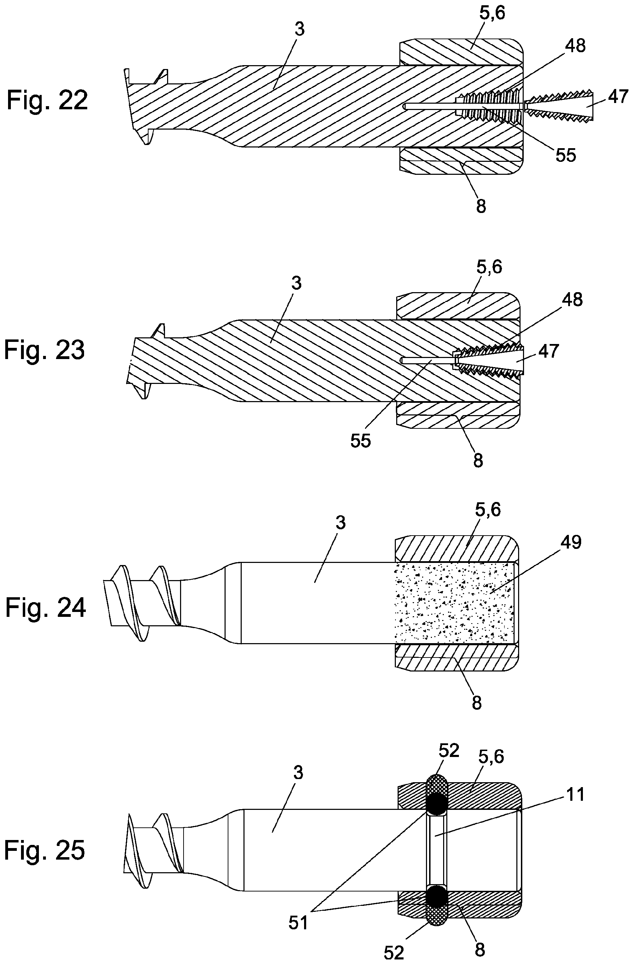

Anhand der Fig. 22 bis 25 werden nachfolgend noch alternative Ausgestaltungsformen erläutert, welche der Fixierung der Implantatschrauben 3 in den entsprechenden Implantataufnahmehülsen 5 bzw. 6 dienen können. In der Variante gemäß der Fig. 22 und 23, welche einen Schnitt durch eine entsprechende Implantatschraube 3 und eine entsprechende Implantataufnahmehülse 5 bzw. 6 zeigen, ist die Fixierungseinrichtung 9 in Form einer Spreizeinrichtung gezeigt. Es handelt sich dabei um einen Spreizkeil 47, welcher in eine entsprechende Spreizkeilaufnahme 48 im Befestigungsabschnitt 8 der Implantatschraube 3 eingeschraubt werden kann, um so den Befestigungsabschnitt 8 der Implantatschraube 3 aufzuweiten und in der Implantataufnahmehülse 5 bzw. 6 auf diese Art und Weise festzuklemmen. Mittels des Schlitzes 55 im Befestigungsabschnitt 8 kann das Aufweiten durch den Spreizkeil 47 erleichtert werden.

Fig. 24 zeigt ein Beispiel einer rein reibschlüssigen Fixierung des Befestigungsabschnitts 8 der Implantatschraube 3 in der entsprechenden Implantataufnahmehülse 5 in einer teilweise geschnittenen Darstellung. Durch die aufgeraute Oberfläche 49 am Befestigungsabschnitt 8 und/oder der korrespondierenden Oberfläche der Implantataufnahmehülse 5 bzw. 6 kommt es bei einer entsprechenden Vorspannung mittels der Expansionseinrichtung 4 zu einem so starken Reibschluss, dass dadurch die Expansionseinrichtung 4 mit seinen Implantataufnahmehülsen 5 und 6 ausreichend fest an den Implantatschrauben 3 befestigt ist. Ein Lösen der Expansionseinrichtung 4 mit ihren Implantataufnahmehülsen 5 und 6 von den Implantatschrauben 3 ist dann nur möglich, wenn durch entsprechende Einstellung der Expansionseinrichtung 4 keine Spannung zwischen Implantataufnahmehülsen 5 und 6 einerseits und Implantatschrauben 3 vorherrscht.

Fig. 25 zeigt in einem Schnitt durch eine Implantataufnahmehülse 5 bzw. 6 und eine Implantatschraube 3 ein Beispiel, wie die Fixierungseinrichtung 9 in Form einer Schnappverbindung ausgeführt sein könnte. In diesem Ausführungsbeispiel befindet sich im Befestigungsabschnitt 8 wiederum eine als umlaufende Nut ausgebildete Formschlusselementaufnahme 11, in die eine mittels des elastischen Elements 52 vorgespannte Rastkugel 51 einrasten und natürlich bei entsprechendem Ziehen an der Implantataufnahmehülse 5 bzw. 6 bzw. an der Expansionseinrichtung 4 auch wieder gelöst werden kann.

### Legende zu den Hinweisziffern:

| | | | |
|---|---|---|---|
| 1 | Distraktorvorrichtung | 28 | Riegel |
| 2 | Kiefer | 29 | Werkzeugaufnahme |
| 3 | Implantatschraube | 30 | erstes Innengewinde |
| 4 | Expansionseinrichtung | 31 | zweites Innengewinde |
| 5 | Implantataufnahmehülse | 32 | Außengewinde |
| 6 | Implantataufnahmehülse | 33 | Außengewinde |
| 7 | Gewindeabschnitt | 34 | Werkzeugeingriff |
| 8 | Befestigungsabschnitt | 35 | Drehwerkzeug |
| 9 | Fixierungseinrichtung | 36 | Mehrkant |
| 10 | Formschlusselement | 37 | Gaumennaht |
| 11 | Formschlusselementaufnahme | 38 | Gingiva |
| | | 39 | Griff |
| 12 | Expansionsgewindestab | 40 | Schwenkrichtung |
| 13 | Expansionsgewindestab | 41 | Abflachung |
| 14 | Expansionsmutter | 42 | Verzahnung |
| 15 | Aufnahmehohlraum | 43 | Zwischenraum |
| 16 | Längsmittelachse | 44 | Winkel |
| 17 | Winkel | 45 | Gerade |
| 18 | Längsmittelachse | 46 | Orthogonale |
| 19 | Setzeinrichtung | 47 | Spreizkeil |
| 20 | Halteplatte | 48 | Spreizkeilaufnahme |
| 21 | Retentionsfläche | 49 | aufgeraute Oberfläche |
| 22 | Zahn | 50 | Schnappverbindung |
| 23 | Führungsstab | 51 | Rastkugel |
| 24 | Führungsfläche | 52 | elastisches Element |
| 25 | Verriegelungseinrichtung | 53 | schwenkbarer Arm |
| | | 54 | Schrägfläche |
| 26 | Führungshülse | 55 | Pfeil |
| 27 | Verriegelungsstab | 56 | Markierung |

## Patentansprüche

1. Distraktorvorrichtung (1) zum kieferorthopädischen Aufweiten eines menschlichen Kiefers (2), insbesondere zur Gaumennahterweiterung, wobei die Distraktorvorrichtung (1) zumindest zwei Implantatschrauben (3) und eine längenverstellbare Expansionseinrichtung (4) und zumindest zwei, an der Expansionseinrichtung (4) befestigte, Implantataufnahmehülsen (5, 6) für jeweils eine der Implantatschrauben (3) aufweist, wobei der Abstand der Implantataufnahmehülsen (5, 6) zueinander mittels Längenverstellung der Expansionseinrichtung (4) veränderbar ist und die Implantatschrauben (3) jeweils einen Gewindeabschnitt (7) zum Einschrauben der jeweiligen Implantatschraube (3) in den Kiefer (2) und einen Befestigungsabschnitt (8) zur Befestigung der jeweiligen Implantatschraube (3) in der jeweiligen Implantataufnahmehülse (5, 6) aufweisen, **dadurch gekennzeichnet, dass** die Implantatschrauben (3) in zumindest einem Betriebszustand der Distraktorvorrichtung (1) über ihre gesamte Länge vollständig durch die jeweilige Implantataufnahmehülse (5, 6) hindurchsteckbar sind.

2. Distraktorvorrichtung (1) nach Anspruch 1, **dadurch gekennzeichnet, dass** die Distraktorvorrichtung (1) für zumindest eine, vorzugsweise für jede Implantataufnahmehülse (5, 6) eine, Fixierungseinrichtung (9) aufweist, wobei der Befestigungsabschnitt (8) der jeweiligen Implantatschraube (3) in einem Fixierungszustand der Fixierungseinrichtung (9) in der jeweiligen Implantataufnahmehülse (5, 6) fixierbar und in einem Freigabezustand der Fixierungseinrichtung (9) freigegeben ist, wobei der Freigabezustand der Betriebszustand ist, in dem die jeweilige Implantatschraube (3) über ihre gesamte Länge vollständig durch die jeweilige Implantataufnahmehülse (5, 6) hindurchsteckbar ist.

3. Distraktorvorrichtung (1) nach Anspruch 2, **dadurch gekennzeichnet, dass** die Fixierungseinrichtung (9) ein Formschlusselement (10) und eine Formschlusselementaufnahme (11) aufweist, wobei das Formschlusselement (10) im Fixierungszustand in die Formschlusselementaufnahme (11) eingreift und im Freigabezustand außerhalb der Formschlusselementaufnahme (11) angeordnet ist.

4. Distraktorvorrichtung (1) nach Anspruch 3, **dadurch gekennzeichnet, dass** das Formschlusselement (10), vorzugsweise schwenkbar, an der jeweiligen Implantataufnahmehülse (5, 6) befestigt ist und die Formschlusselementaufnahme (11) am Befestigungsabschnitt (8) der jeweiligen Implantatschraube (3), vorzugsweise als umlaufende Nut, ausgebildet ist.

5. Distraktorvorrichtung (1) nach Anspruch 4, **dadurch gekennzeichnet, dass** das Formschlusselement (10) in Richtung hin zum Fixierungszustand elastisch vorgespannt ist.

6. Distraktorvorrichtung (1) nach einem der Ansprüche 1 bis 5, **dadurch gekennzeichnet, dass** die Expansionseinrichtung (4) zwei Expansionsgewindestäbe (12, 13) und eine Expansionsmutter (14) aufweist, wobei die Expansionsgewindestäbe (12, 13) jeweils an zumindest einer der Implantataufnahmehülsen (5, 6) fixiert und auf einander gegenüberliegenden Seiten in die Expansionsmutter (14) eingeschraubt oder einschraubbar sind.

7. Distraktorvorrichtung (1) nach Anspruch 6, **dadurch gekennzeichnet, dass** einer der Expansionsgewindestäbe (12) einen Aufnahmehohlraum (15) aufweist, in den der andere der Expansionsgewindestäbe (13) einführbar ist.

8. Distraktorvorrichtung (1) nach einem der Ansprüche 1 bis 7, **dadurch gekennzeichnet, dass** die, oder zumindest zwei der, Implantataufnahmehülsen (5, 6) auf einander gegenüberliegenden Seiten der Expansionseinrichtung (4) angeordnet sind und/oder Längsmittelachsen (16) der auf einander gegenüberliegenden Seiten der Expansionseinrichtung (4) angeordneten Implantataufnahmehülsen (5, 6) einen Winkel (17) im Bereich von 100° bis 105° mit einer Längsmittelachse (18) der Expansionseinrichtung (4) einschließen, wobei die Expansionseinrichtung (4) entlang ihrer Längsmittelachse (18) längenverstellbar ist.

9. Anordnung mit einer Distraktorvorrichtung (1) nach einem der Ansprüche 1 bis 8, wobei die Anordnung zusätzlich eine Setzeinrichtung (19) mit einer Halteplatte (20) mit Retentionsflächen (21) zur Befestigung der Halteplatte (20) an im Kiefer (2) angeordneten Zähnen (22) und mit zumindest zwei Führungsstäben (23) aufweist und die Distraktorvorrichtung (1) zu den Führungsstäben (23) korrespondierende Führungsflächen (24) aufweist, wobei die Distraktorvorrichtung (1) für ihre Positionierung relativ zum Kiefer (2) entlang der Führungsstäbe (23) verschiebbar geführt ist.

10. Anordnung nach Anspruch 9, **dadurch gekennzeichnet, dass** die Setzeinrichtung (19) eine Verriegelungseinrichtung (25) zum, vorzugsweise formschlüssigen, lösbaren Verriegeln der Distraktorvorrichtung (1) in der relativ zum Kiefer (2) eingestellten Position an der Setzeinrichtung (19) aufweist.

## Claims

1. A distractor device (1) for orthodontic widening of a human jaw (2), in particular for palatal expansion, wherein the distractor device (1) has at least two implant screws (3) and an adjustable-length expansion means (4), and at least two implant receiving sleeves (5, 6), fastened to the expansion means (4), for one of the implant screws (3) in each case, wherein the distance of the implant receiving sleeves (5, 6) from each other is changeable by means of adjustment of the length of the expansion means (4), and the implant screws (3) in each case have a threaded portion (7) for screwing the respective implant screw (3) into the jaw (2) and a fastening portion (8) for fastening the respective implant screw (3) in the respective implant receiving sleeve (5, 6), **characterised in that** the implant screws (3) in at least one operating state of the distractor device (1) can be pushed completely through the respective implant receiving sleeve (5, 6) over their entire length.

2. A distractor device (1) according to claim 1, **characterised in that** the distractor device (1), for at least one, preferably for each, implant receiving sleeve (5, 6), has a fixing means (9), the fastening portion (8) of the respective implant screw (3) in a fixing state of the fixing means (9) being able to be fixed in the respective implant receiving sleeve (5, 6) and being released in a release state of the fixing means (9), the release state being the operating state in which the respective implant screw (3) can be pushed completely through the respective implant receiving sleeve (5, 6) over its entire length.

3. A distractor device (1) according to claim 2, **characterised in that** the fixing means (9) has a form-fitting element (10) and a form-fitting element receptacle (11), the form-fitting element (10) in the fixing state engaging in the form-fitting element receptacle (11) and in the release state being arranged outside the form-fitting element receptacle (11).

4. A distractor device (1) according to claim 3, **characterised in that** the form-fitting element (10) is fastened, preferably pivotably, on the respective implant receiving sleeve (5, 6), and the form-fitting element receptacle (11) is formed on the fastening portion (8) of the respective implant screw (3), preferably as a circumambient groove.

5. A distractor device (1) according to claim 4, **characterised in that** the form-fitting element (10) is elastically pre-tensioned in the direction towards the fixing state.

6. A distractor device (1) according to one of claims 1 to 5, **characterised in that** the expansion means (4) has two threaded expansion rods (12, 13) and an expansion nut (14), the threaded expansion rods (12, 13) being fixed in each case to at least one of the implant receiving sleeves (5, 6) and being screwed in or able to be screwed in to the expansion nut (14) on opposing sides.

7. A distractor device (1) according to claim 6, **characterised in that** one of the threaded expansion rods (12) has a receiving cavity (15) into which the other of the threaded expansion rods (13) can be introduced.

8. A distractor device (1) according to one of claims 1 to 7, **characterised in that** the, or at least two of the, implant receiving sleeves (5, 6) are arranged on opposing sides of the expansion means (4), and/or longitudinal centre lines (16) of the implant receiving sleeves (5, 6) arranged on opposing sides of the expansion means (4) enclose an angle (17) in the range from 100° to 105° with a longitudinal centre line (18) of the expansion means (4), the expansion means (4) being adjustable lengthwise along its longitudinal centre line (18).

9. An arrangement having a distractor device (1) according to one of claims 1 to 8, wherein the arrangement additionally has a setting means (19) with a holding plate (20) with retention surfaces (21) for securing the holding plate (20) to teeth (22) arranged in the jaw (2) and with at least two guide rods (23), and the distractor device (1) has guide surfaces (24) corresponding to the guide rods (23), the distractor device (1) for its positioning relative to the jaw (2) being guided displaceably along the guide rods (23).

10. An arrangement according to claim 9, **characterised in that** the setting means (19) has a locking means (25) for the, preferably form-fitting, detachable locking of the distractor device (1) in the position set relative to the jaw (2) on the setting means (19).

## Revendications

1. Dispositif distracteur (1) pour l'élargissement orthodontique d'une mâchoire humaine .(2), en particulier pour l'élargissement du raphé du palais, le dispositif distracteur (1) étant muni d'au moins deux vis d'implant (3) et d'un dispositif d'expansion (4) réglable en longueur et d'au moins deux douilles de réception d'implant (5, 6) fixées sur le dispositif d'expansion (4), une pour chaque vis d'implant (3), la distance des douilles de réception d'implant (5, 6) l'une par rapport à l'autre pouvant être modifiée au moyen d'un réglage en longueur du dispositif d'expansion (4), et les vis d'implant (3) étant munies chacune d'une section filetée (7) pour le vissage de la vis d'implant considérée (3) dans la mâchoire (2), et d'une section de fixation (8) pour la fixation de la vis d'implant considérée (3) dans la douille de réception d'implant respective (5, 6), **caractérisé en ce que** les vis d'implant. (3) peuvent être entièrement enfoncées sur toute leur longueur à travers la douille de réception d'implant respective (5, 6) dans au moins un état de fonctionnement du dispositif distracteur (1).

2. Dispositif distracteur (1) selon la revendication 1, **caractérisé en ce que** le dispositif distracteur (1) est muni d'un dispositif de fixation (9) pour au moins une, de préférence pour chaque douille de réception d'implant (5, 6), la section de fixation (8) de la vis d'implant respective (3) pouvant être fixée, dans un état de fixation du dispositif de fixation (9), dans la douille de réception d'implant respective (5, 6), et étant libérée dans un état de libération du dispositif de fixation (9), l'état de libération étant l'état de fonctionnement dans lequel la vis d'implant respective (3) peut être entièrement insérée sur toute sa longueur à travers la douille de réception d'implant respective (5, 6).

3. Dispositif distracteur (1) selon la revendication 2, **caractérisé en ce que** le dispositif de fixation (9) est muni d'un élément de liaison par complémentarité de forme (10) et d'un logement d'élément de liaison par complémentarité de forme (11), l'élément de liaison par complémentarité de forme (10) s'engageant dans le logement d'élément de liaison par complémentarité de forme (11) à l'état de fixation et étant disposé à l'extérieur du logement d'élément de liaison par complémentarité de forme (11) à l'état de libération.

4. Dispositif distracteur (1) selon la revendication 3, **caractérisé en ce que** l'élément de liaison par complémentarité de forme (10) est fixé, de préférence de manière pivotante, sur la douille de réception d'implant respective (5, 6), et le logement d'élément de liaison par complémentarité de forme (11) est réalisé sur la section de fixation (8) de la vis d'implant respective (3), de préférence sous la forme d'une rainure périphérique.

5. Dispositif distracteur (1) selon la revendication 4, **caractérisé en ce que** l'élément de liaison par complémentarité de forme (10) est précontraint de manière élastique en direction de l'état de fixation.

6. Dispositif distracteur (1) selon l'une des revendications 1 à 5, **caractérisé en ce que** le dispositif d'expansion (4) est muni de deux tiges filetées d'expansion (12, 13) et d'un écrou d'expansion (14), les tiges filetées d'expansion (12, 13) étant fixées chacune à au moins l'un des manchons de réception d'implant (5, 6) et étant vissées ou vissables dans l'écrou d'expansion (14) sur des côtés opposés.

7. Dispositif distracteur (1) selon la revendication 6, **caractérisé en ce que** l'une des tiges filetées d'expansion (12) est munie d'une cavité de réception (15) dans laquelle peut être introduite l'autre des tiges filetées d'expansion (13).

8. Dispositif distracteur (1) selon l'une des revendications 1 à 7, **caractérisé en ce que** les douilles de réception d'implant (5, 6), ou au moins deux d'entre elles, sont disposées sur des côtés opposés du dispositif d'expansion (4), et/ou des axes médians longitudinaux (16) des douilles de réception d'implant (5, 6) disposées sur des côtés opposés du dispositif d'expansion (4), 6) forment avec un axe central longitudinal (18) du dispositif d'expansion (4) un angle (17) dans la plage de 100° à 105°, le dispositif d'expansion (4) étant réglable en longueur le long de son axe central longitudinal (18).

9. Agencement avec un dispositif distracteur (1) selon l'une des revendications 1 à 8, dans lequel l'agencement est muni en outre d'un dispositif de mise en place (19) avec une plaque de maintien (20) ayant des surfaces de rétention (21) pour la fixation de la plaque de maintien (20) sur des dents (22) disposées dans la mâchoire (2), et avec au moins deux tiges de guidage (23), et le dispositif distracteur (1) est muni de surfaces de guidage (24) correspondant aux tiges de guidage (23), le dispositif distracteur (1) étant guidé de manière mobile le long des tiges de guidage (23) pour son positionnement par rapport à la mâchoire (2).

10. Agencement selon la revendication 9, **caractérisé en ce que** le dispositif de mise en place (19) est muni d'un dispositif de verrouillage (25) pour verrouiller de façon réversible sur le dispositif de pose (19), de préférence par complémentarité de formes, le dispositif distracteur (1) dans la position réglée par rapport à la mâchoire (2).
